# EUROPÄISCHE PATENTSCHRIFT

(11) **EP 0 965 348 B1**
(45) Veröffentlichungstag und Bekanntmachung des Hinweises auf die Patenterteilung: **19.07.2000**
(21) Anmeldenummer: 98107294.5
(22) Anmeldetag: 22.04.1998
(51) Int. Cl.: A61K 35/78

(54) **Verfahren zur schonenden Gewinnung von Extraktfraktionen aus Hypericum perforatum L., diese enthaltende pharmazeutische Zubereitungen und Verwendung derselben**
Process for gently recovering extract fractions from Hypericum perforatum L., pharmaceutical compositions containing them and the use thereof
Procédé d'obtention soignée de fractions d'extrait d'Hypericum perforatum L., les compositions pharmaceutiques qui les contiennent ainsi que leur utilisation

(43) Veröffentlichungstag der Anmeldung: 22.12.1999
(73) Patentinhaber: Bionorica Arzneimittel GmbH, 92318 Neumarkt (DE)
(72) Erfinder: Joseph, Heinz Walter, 92348 Berg (DE)
(74) Vertreter: Schwabe - Sandmair - Marx

(56) Entgegenhaltungen:
- EP-A- 0 826 372
- WO-A-97/13489
- DE-A- 3 935 772
- B. MEIER: "THE EXTRACTION STRENGTH OF ETHANOL/WATER MIXTURES COMMONLY USED FOR THE PROCESSING OF HERBAL DRUGS" PLANTA MEDICA, Bd. 57, Nr. 8 SUPPLEMENT, Dezember 1991, Seiten A26-A27, XP002069894
- DATABASE WPI Section Ch, Week 9344 Derwent Publications Ltd., London, GB; Class B04, AN 93-348326 XP002069895 & JP 05 255 046 A (KAO CORP)

## Beschreibung

Die vorliegende Erfindung betrifft ein Verfahren zur schonenden Gewinnung von Extraktfraktionen aus Hypericum perforatum L. (deutsch: Johanniskraut) mit einem hohen Anteil an Wirk- und/oder Leitsubstanzen und diese Extraktfraktionen enthaltende pharmazeutische Zubereitungen sowie deren Verwendung zur Therapie von Depressionen.

Das Johanniskraut, Hypericum perforatum L., gehört zu den schon seit langem genutzten Arzneipflanzen zur Behandlung psycho-vegetativer Störungen (Daunderer, Klinische Toxikologie, 81. Erg.Lfg 3/94). Weiterhin wird Hypericum perforatum bei photosensiblen Dermatosen, funktionellen und arteriosklerotischen Depressionen, Zuständen nach Commotio Cerebri und Rückenmarkserschütterungen angewandt (Roth, Daunderer, Giftliste, 60. Erg.Lfg. 10/98). Das Oleum Hyperici wird zur Behandlung von Verbrennungen und Wunden verwendet. Weiterhin bekannt ist die antimikrobioelle Aktiviät von Hypericum gegen Staphylokokken und Streptokokken (Roth, Daunderer, aaO.) oder Viren (EP-A-0 256 452). Als Droge werden das getrocknete, kurz vor oder während der Blütezeit gesammelte Kraut bzw. wässrige oder alkoholische, vor allem ethanolische Gesamtextrakte oder Gesamttrockenextrake verwendet, siehe beispielsweise Helarium® Hypericum.

Im einzelnen wurden aus Hypericum ca. 15 Wirksubstanzen isoliert, von denen vor allem das Hypericin, das Pseudohypericin, das Hyperforin, ein prenyliertes Phloroglucin, das Adhyperforin und das Hyperosid zu nennen sind.

Die antidepressive Wirkung wurde bislang dem Hypericin als die Wirksamkeit bestimmendem Inhaltsstoff zugeschrieben (siehe Roth, Daunderer, aaO.; Daunderer, aaO.). Hierzu wurde angenommen, daß der Wirkmechanismus auf einer Hemmung der MAO (Monoamminooxidase) oder der COMT (Catechol-O-Methyltransferase) beruht. Da eine entsprechende Wirkung bei Verabreichung von freiem Hypericin nicht bestätigt werden konnte, ist in der Literatur ein Streit darüber entstanden, ob die Wirkung nicht doch dem Hyperforin als weiterer Wirksubstanz zuzuschreiben sei (siehe beispielsweise die EP-A-0 599 307).

Im Handel befindlich sind bislang Gesamttrockenextrakte von Hypericum sowie ein in Bezug auf das Hyperforin angereicherter Trockenextrakt, der gemäß dem Verfahren der EP-A-0 599 307 hergestellt wurde. Alle Präparate sind hinsichtlich ihrer Wirkung durchaus mit den tricyclischen Antidepressiva vergleichbar, weisen jedoch erheblich verringerte Nebenwirkungen, insbesondere bezüglich der Verträglichkeit, der Reaktionsgeschwindigkeit, der Müdigkeit und der Fahrtüchtigkeit auf. Sie stellen damit ein im Vergleich zu den tricyclischen Antidepressiva für den Patienten erheblich tolerableres Therapeutikum dar bei gleicher Wirksamkeit. Das Produkt hat eine erheblich bessere Patienten-Compliance.

Hypericin und/oder Hyperforin enthaltende Extrakte von Hypericum bzw. die entsprechenden Trockenextrakte können nach verschiedenen bekannten Aufbereitungsarten und Trocknungsverfahren hergestellt werden. Dabei wird in der Regel von einem Gesamtextrakt ausgegangen. Dessen Zusammensetzung ist jedoch durch das gewählte Ausgangsmaterial bestimmt. Die Steuerung des Substanzgehalts ist daher auf die Auswahl geeigneter Pflanzen oder die Aufbereitungsart z.B. die Wahl des Extraktionslösungsmittels bestimmt, da Hyperforin extrem empfindlich und leicht zersetzlich ist. Hypericin wird als Reinsubstanz beispielsweise gemäß der DE-B-15 69 849 erhalten.

Leider gelingt es mittels der im Stand der Technik bekannten Verfahren wie der reinen Lösungsmittelextraktion (DE-A-15 69 849 oder DE-C-39 35 772) sowie der zweistufigen Extraktion (EP-A-0 599 307) nicht, Hypericin- und/oder Hyperforin-haltige Extrakte mit definiertem und einstellbarem Wirkstoffgehalt zu gewinnen. Um insbesondere Hypericin-haltige Extrakte herzustellen, bedient man sich der Methode eines lipophilen Auszugs. Diese lipophilen Auszüge können durch entsprechende Lösungsmittel (Methanol, Aceton, Methylethylketon) gewonnen werden. Daneben können zur Gewinnung der Hyperforin-haltigen Extrakte zunächst wässrige oder hydrophile Auszüge hergestellt werden, die anschließend nochmals mit lipophilen Lösungsmitteln wie DMSO oder Dioxan ausgewaschen oder einer lipophilen Feststoffextraktion z.B. an PVP oder Aktivkohle unterworfen werden (EP-A-0 599 307 oder EP-A-0 702 957).

Der Nachteil dieser Verfahren besteht darin, daß das lipophilere Lösungsmittel durch für die pharmazeutische Applikation geeignete Lösungsmittel ersetzt werden muß, die Extraktgewinnung ein zweistufiges Verfahren erfordert und zudem durch die Varianz der Pflanzen bezüglich des Wirkstoffgehalts ebenfalls variable Inhaltsstoffspektren entstehen, diese aber nicht frei steuerbar, sondern eben vom Gesamtwirkstoffgehalt abhängig sind. Bei der Herstellung des Trockenextraktes gemäß dem Stand der Technik gelingt es also nicht, steuernd auf die Inhaltsstoffe einzuwirken. Dies ist allenfalls durch die Wahl eines weiteren Lösungsmittels oder Auswahl des pflanzlichen Ausgangsmaterials möglich.

Bei den ebenfalls bekannten einfachen ethanolischen Gesamttrockenextrakten, die mittels Sprühtrocknung und konventioneller Vakuumtrocknung hergestellt werden, kann ebenfalls nicht steuernd auf das Inhaltsspektrum eingewirkt werden, da im Trockenextrakt oder der Trockenextraktzubereitung alle ursprünglich vorhandenen und vorab extrahierten Inhaltsstoffe vorliegen. Eine Steuerung des Inhaltstoffsspektrums ist ebenfalls nur durch die Auswahl geeigneter Pflanzen möglich.

Aufgabe der Erfindung war es daher, ein möglichst einfaches, schonendes, aber in Bezug auf den Wirkstoffgehalt (Hypericin und/oder Hyperforin) steuerbares Extraktionsverfahren zu entwickeln, das den Erhalt bezüglich des Hyperforin- und Hypericingehalts definierter und einstellbarer Extrakte gestattet.

Gelöst wird diese Aufgabe durch ein Verfahren zur Gewinnung von Extraktfraktionen von Hypericum perforatum L., bei dem man
a. gemäß bekannter Verfahren einen wäßrigen ethanolischen Extrakt von Hypericum perforatum L. gewinnt,
b. den Extrakt auf einen vorgewählten Konzentrationsgrad einengt, und
c. das in Schitt b. ausgefallene Präzipitat von Überstand abtrennt,
wobei die Schritte b. und c. mit dem jeweiligen Überstand aus Schritt c. mindestens einmal wiederholt werden.

Vorzugsweise weist das Extraktionslösungsmittel zur Gewinnung des wäßrigen ethanolischen Extrakts in Schritt a. einen Ethanolgehalt von mindestens 40 Vol.- %, stärker bevorzugt 60 - 90 Vol.- %, am meisten bevorzugt 80 Vol.- % auf.

Vorzugsweise werden die Schritte b. und c. mindestens einmal, stärker bevorzugt 2 bis 4 mal und am meisten bevorzugt 2 mal wiederholt. Damit werden mindestens 2, vorzugsweise 2 bis 4 oder 5 und am meisten bevorzugt 3 oder 4 Extraktfraktionen erhalten, wobei die höhere Zahl an Extraktfraktionen erhalten wird, wenn in der letzten Schrittfolge b./c. im Schritt b. nicht auf einen Konzentrationsgrad von 100 % eingeengt und so neben dem Präzipitat ein (Rest)Überstand erhalten wird.

Gemäß einer bevorzugten Ausführungsform umfaßt das Verfahren weiterhin einen Schritt d., bei dem man das in Schritt c. erhaltene Präzipitat rücklöst. Die Rücklösung erfolgt vorzugsweise in einem Wasser/Ethanol-Gemisch mit höherem Gehalt an Ethanol als das ursprüngliche Extraktionslösungsmittel, beispielsweise einem Mischverhältnis Ethanol/Wasser von 95 bis 50 / 5 bis 50, stärker bevorzugt 90 bis 80 / 20 bis 10 (Vol./Vol.). Zur Verfeinerung der Trennung können die rückgelösten Präzipitate wiederum einer oder mehreren Schrittfolge(n) (b. - d.) unterworfen und so nochmals fraktioniert werden.

Vorzugsweise werden Präzipitate bzw. Fraktionen gewonnen, die Konzentrationgraden, berechnet in Redestillatmenge (g): Ausgangsmenge (g), von 0 bis 40-50 %, 40-50 bis 60-75 % und 60-75 bis 100 % entsprechen. Soll eine feinere Trennung erzielt werden, können selbstverständlich weitere Fraktionen beispielsweise entsprechend Konzentrationsgraden von null bis 40 %, 40-50 %, 50-65 %, 65-75 % und 75-85 % aufgefangen werden. Noch weitere Trennungen sind denkbar.

Aufgrund seines lipophileren Charakters fällt bis zu Konzentrationsgraden von ca. 50 % beim Eindampfen oder Konzentrieren zunächst das Hyperforin aus, während das Hypericin als hydrophilere Substanz bis zu einem Eindampfungsgrad von ca. 50 % überwiegend in Lösung verbleibt. Ab Konzentrationsgraden von ca. 50 % steigt der Hypericingehalt des Präzipitats deutlich an, wobei bei Konzentrationsgraden von ca. 80 bis 85 % gegenüber dem Gesamtextrakt angereicherte Hypericinfraktionen im Präzipitat erhalten werden. Demgegenüber ist das anfänglich erhaltende Präzipitat gegenüber dem Gesamtextrakt an Hyperforin angereichert.

Durch Wahl der jeweiligen Konzentrationsgrade und Aufteilung der Fraktionierung sowie gegebenenfalls durch Rücklösen und erneute Fraktionierung kann somit gezielt Hyperforin bzw. Hypericin an- oder abgereichert werden. Der Grad der Anreicherung und damit der Wirkstoffgehalt ist daher durch geeignete Wahl der Schrittzahl und Konzentrationsgrade im erfindungsgemäßen Verfahren frei einstellbar.

Vorteilhafterweise ist bei dem erfindungsgemäßen Verfahren lediglich ein Auszugsmittel, das den gesetzlichen Vorschriften entspricht, erforderlich, nämlich eine wäßrige ethanolische Lösung. Nicht anzuwenden sind weitere, lipophilere Extraktionsmittel wie beispielsweise Essigsäuerethylester, Aceton, Methylethylketon oder chlorierte Kohlenwasserstoffe. Ebenfalls nicht erforderlich sind Schritte der lipophilen Feststoffextration unter Anwendung beispielsweise von Aktivkohle oder PVP. Da weitere Lösungsmittel oder Extraktionsmittel vermieden werden können, führt dies gleichzeitig zur wirtschaftlichen und ökologischen Vorteilen. Weiterhin kann der beim Konzentrieren abgezogene Ethanol bzw. das abgezogene Ethanol/Wasser-Gemisch rückgewonnen und wieder in das Verfahren eingesetzt werden, wodurch sich erhebliche Einsparungen bezüglich des Extraktionsmittels ergeben.

Aufgrund der schonenden Eindämpfung/Fraktionierung wird eine Zersetzung des empfindlicheren Hyperforins im wesentlichen vermieden, was ebenfalls als Vorteil gegenüber dem Stand der Technik zu sehen ist.

Belastung des Fluidextrakts wie oben angedeutet durch lipophile bzw. hydrophile Lösungsmittel entfällt ebenso wie andere Extraktions- bzw. Chromatographieaufbereitungen. Nachgeschaltete Aufbereitungsarten kommen mit einem weitaus geringeren bzw. anderen Aufbereitungsmaterialen aus. Wesentlichster Vorteil ist jedoch, daß das vorliegende schonende Verfahren die Möglichkeit gibt, steuernd auf den Gehalt der Extrakte an den jeweiligen Wirkstoffen einzuwirken. Dieses war bislang nicht möglich.

Die Konzentrierung bzw. Fraktionierung der Schritte b. und c. kann mit Hilfe jeglicher Vorrichtung beispielsweise einem Rotationsverdampfer erfolgen, die eine gezielte Steuerung des Konzentrationsgrades gestattet. Beispielsweise kann sie mit Hilfe einer Inox-Glatt®-Anlage ausgeführt werden, wie den Trocknungssystemen Inox-Glatt IUT 20, IUT 50, IUT 100 oder IUT 2000.

Die Trocknung erfolgt bei einer Vor- bzw. Rücklauftemperatur zwischen 150 - 100 °C bzw. 20 - 1°C, vorzugsweise 120 °C bzw. 5 °C, einer Innenraumtemperatur zwischen 10 °C und 80°C, bevorzugt zwischen 25 °C und 70 °C sowie einem Druck zwischen 0,5 und 1000 mbar, bevorzugt zwischen 5 und 100 mbar, besonderes bevorzugt zwischen 30 und 70 mbar. Das Rührwerk wird zur Trocknung auf eine Geschwindigkeit zwischen 0 und 10 Umdrehungen pro Minute (Upm), bevorzugt 0 und 5 Upm und der Zerhacker auf eine Zerhackergeschwindigkeit zwischen 200 und 800 Upm eingestellt.

Zu Beginn der Aufkonzentration wird darauf geachtet, daß bei der eingestellten Vorbzw. Rücklauftemperatur und einer Innentemperatur zwischen 10 und 80 °C , sowie einer Brüdentemperatur zwischen 15 und 65 °C, vorzugsweise zwischen 25 und 55 °C das lipophile Medium - in diesem Fall Ethanol - abgezogen wird.

Am Beispiel der Herstellung von Hypericum-Trockenextrakten konnte nachgewiesen werden, daß im Vergleich zu den gängigen Verfahren ein weitaus höherer Gesamtgehalt aller Extraktfraktionen an Hyperforin bzw. an Hypericin erzielt wurde. Dieser Befund war völlig überraschend, da im Bereich des eingesetzten Extraktionsmittels hinsichtlich des lipophilen Verhaltens der Verbindungen keine Unterschiede auftraten und die enthaltenen Grenzkonzentrationen sich ohne Schwierigkeiten im vorgelegten Extraktionsmittel lösten.

Durch die Behandlung der flüssigen Extrakte nach dem erfindungsgemäßen Verfahren gelang außerdem die bisher nicht mögliche, gewillkürte Einstellung des Wirkstoffgehalts, ebenso wie die bisher schonendste Aufkonzentration von Trockenextrakten mit definiertem hohen Gehalt an Leit- bzw. Wirkstoffen aus Drogen, die mit einer bestimmten Ethanol/Wasser-Konzentration hergestellt wurden. Aufgrund des definierten Wirkstoffgehalts vereinfacht sich zudem die Aufarbeitung der Extraktfraktionen.

Alle übrigen bekannten Trocknungsverfahren ergeben in ihrer physischen Aufbereitung schlechter steuerbare und in ihrem Wirkstoffgehalt festgelegte Trockenextrakte. Damit verbunden ist eine Reduktion des möglichen therapeutischen Erfolges.

Auch die schonende Zurückgewinnung von Lösungsmitteln, die einer weiteren Verwendung zugeführt werden können, ist ein weiterer ökologisch vorteilhafter Aspekt dieses Verfahrens. Schließlich ist als unerwarteter Vorteil anzusehen, daß die gewonnenen Extraktfraktionen einer weiteren Konzentration oder Trennung unterzogen werden können, um daraus wertvolle Naturstoffe zu gewinnen.

Die Präzipitate bzw. Überstände als Produkte können zu fertigen pharmazeutischen Zubereitungen oder Halbfabrikaten weiterverarbeitet werden. Dies kann im gleichen Trocknungsgerät und in ihrer angereicherten Form geschehen.

Die pharmazeutischen Zubereitungen können in zur oralen oder parenteralen Applikation geeigneter Form beispielsweise in Form von Lösungen, insbesondere Tinkturen oder Trinklösungen, Suspensionen, Kapseln, Dragees, Tabletten, Suppositorien etc. vorliegen. Sie können übliche geeignete pharmazeutische Hilfs- und Trägerstoffe, wie Füllstoffe, Granulierhilfen, Süßstoffe, Aromen, Konservierungsmittel etc. enthalten.

Die pharmazeutischen Zubereitungen, die sowohl in Bezug auf das Hyperforin als auch das Hypericum angereicherte Extraktfraktionen enthalten können, werden vorzugsweise zur Behandlung von Depressionen verwendet. Sie können aber genauso für die weiteren Indikationen, die für Hypericum bekannt sind, angewandt werden.

Das vorliegende Beispiel soll die Erfindung lediglich veranschaulichen, es ist nicht als beschränkend aufzufassen.

### Beispiel:

Zur Herstellung der Extraktfraktionen wurden verschiedene Chargen von Hypericum-Tinktur (60/40 EtOH/H₂O-Gemisch) eingesetzt. Bei den Ausgangslösungen wurde der Trockenextraktwirkstoffgesamtgehalt und der Hypericingehalt nach DAC bestimmt und ein Gesamtspektrum über HPLC erstellt. Die interessierenden Verbindungen wurden bei einzelnen Aufkonzentrationsstufen gemessen und abgetrennt. Für das erfindungsgemäße Verfahren wurden mehrere Versuche durchgeführt. Hierbei variierte die Menge der eingesetzten Tinkturen/Fluidextrakte zwischen 50 und 1.000 Litern. Die Tinkturen wurden im Vakuumtrockner der Firma Inox-Glatt bei Raumtemperatur eingesaugt und zwischen 120 °C und 5°C Vorlauf- und Rücklauftemperatur getrocknet.

Die Verdampfungsleistung lag zwischen 300 und 330 Litern pro Stunde. Sie unterliegt Schwankungen aufgrund der anfänglich leicht zu destillierenden Alkoholmenge im Vergleich zu schwerer flüchtigem Wasser. Die Innentemperatur betrug 30-40 °C, die Brüdentemperatur 30-40 °C. Der Druck lag unter 120 mbar.

**Tabelle 1a**

| Konz.-grad in % | spez. Hypericin in % | spez. Hyperforin in % |
|---|---|---|
| 0 | 0,58 | 0,74 |
| 24,7 | 0,26 | 1,6 |
| 32,5 | 0,30 | 1,5 |
| 48,9 | 0,40 | 20,4 |
| 57,9 | 0,73 | 7,1 |
| 66,8 | 1,06 | 1,2 |
| 86,4 | 0,53 | n.n. |

**Tabelle 1b**

| Konz.-grad in % | spez. Hypericin in % (Ausfällungen) | spez. Hyperforin in % (Ausfällungen) |
|---|---|---|
| 0 | 0,52 | 0,74 |
| 32 | 0,25 | 1,6 |
| 49 | 0,40 | 6,1 |
| 59 | 0,73 | 7,4 |
| 67 | 0,78 | 6,1 |
| 76 | 0,94 | 0,4 |
| 85 | 0,83 | 0,4 |
| 94 | 0,48 | n.n. |

Wie die vorstehenden Tabellen zeigen, wird durch das erfindungsgemäße Verfahren im Präzipitat im Vergleich zur Gesamttrocknung anfänglich ein erhöhter Gehalt an Hyperforin festgestellt. Dieses kann nach Ablassen des Überstandes in 90 %-igem Ethanol aus dem Behältnis gelöst und bei Bedarf weiterverwendet werden. Der rückzupumpende Überstand kann weiter aufkonzentriert werden und zeigt in weiteren Präzipitaten im Verlauf der weiteren Aufkonzentrierung einen höheren Gehalt an hydrophilen Inhaltsstoffen, insbesondere Hypericin.

## Patentansprüche

1. Verfahren zur Gewinnung von Extraktfraktionen von Hypericum perforatum L., bei dem man
a) gemäß bekannter Verfahren einen wäßrigen ethanolischen Extrakt von Hypericum perforatum L. gewinnt,
b) den Extrakt auf einen vorgewählten Konzentrationsgrad einengt und
c) das in Schritt b. ausgefallene Präpizitat vom Überstand abtrennt,
wobei die Schritte b. und c. mit dem jeweiligen Überstand aus Schritt c. mindestens einmal wiederholt werden.

2. Verfahren gemäß Anspruch 1, dadurch gekennzeichnet, daß der ethanolische Extrakt einen Ethanolgehalt von mindestens 40 Vol.- % aufweist.

3. Verfahren gemäß Anspruch 2, dadurch gekennzeichnet, daß der Ethanolgehalt 60 bis 90 Vol.- % beträgt.

4. Verfahren gemäß einem der Ansprüche 1-3 bei dem die Schritte b. und c. zweimal bis viermal wiederholt werden.

5. Verfahren gemäß einem der vorstehenden Ansprüche, umfassend zusätzlich einen Schritt d., bei dem man das in Schritt c. erhaltene Präzipitat rücklöst.

6. Verfahren gemäß Anspruch 5, bei dem die Rücklösung in einem Ethanol/Wasser-Gemisch mit höherem Ethanolgehalt als demjenigen des ursprünglichen Extraktionslösungsmittels erfolgt.

7. Verfahren gemäß Anspruch 5 oder 6, bei dem die rückgelösten Präzipitate wiederum einer oder mehreren Schrittfolgen b., c. und wahlweise d. unterworfen werden.

8. Verfahren gemäß einem der vorstehenden Ansprüche, dadurch gekennzeichnet, daß die konzentrierung bzw. Fraktionierung de Schritte b. und c. mit Hilfe einer Inox-Glatt®-Anlage ausgeführt wird.

9. Verfahren gemäß Anspruch 8, dadurch gekennzeichnet, daß die Trocknung bei einer Vor- bzw. Rücklauftemperatur zwischen 150 bis 100 °C bzw. 20 bis 1 °C, einer Innenraumtemperatur zwischen 10 °C und 80°C, einer Brüdentemperatur zwischen 15 und 65 °C, einem Druck zwischen 0,5 und 1000 mbar, einer Rührwerksgeschwindigkeit zwischen 0 und 10 Upm und einer Zerhackergeschwindigkeit zwischen 200 bis 800 Upm geführt wird.

10. Extraktfraktion von Hypericum perforatum L., erhältlich nach einem der Verfahren gemäß Anspruch 1 bis 9.

11. Extraktfraktion gemäß Anspruch 10, dadurch gekennzeichnet, daß es sich um das Präzipitat oder das rückgelöste Präzipitat der ersten Schrittfolge b. bis c. bzw. d. handelt, wobei in Schritt b. bis zu einem Konzentrationsgrad von 40 bis 50% eingeengt wird.

12. Extraktfraktion gemäß Anspruch 10, dadurch gekennzeichnet, daß es sich um das Präzipitat des dritten Schrittes c. bzw. das rückgelöste Präzipitat des dritten Schrittes d. handelt, wobei der dritte Schritt von einem Konzentrationsgrad von 60 bis 75 % auf einen Konzentrationsgrad von 100 % erfolgt.

13. Extrakfraktion gemäß Anspruch 10, dadurch gekennzeichet, daß es sich um eine hinsichtlich des Hypericingehaltes gegenüber dem Gesamtextrakt angereicherte Fraktion handelt.

14. Extraktfraktion gemäß Anspruch 10, dadurch gekennzeichnet, daß es sich um eine hinsichtlich des Hyperforingehaltes gegenüber dem Gesamtextrakt angereicherte Fraktion handelt.

15. Pharmazeutische Zubereitung, enthaltend eine Extraktfraktion gemäß einem der Ansprüche 10 bis 14.

16. Pharmazeutische Zubereitung, enthaltend eine bezüglich des Hypericingehaltes angereicherte Extraktfraktion gemäß Anspruch 13.

17. Pharmazeutische Zubereitung, enthaltend eine bezüglich des Hyperforingehaltes angereicherte Extraktfraktion gemäß Anspruch 14.

## Claims

1. Process for obtaining extract fractions from *Hypericum perforatum* L., in which
a) an aqueous ethanolic extract of *Hypericum perforatum L.* is obtained by known processes,
b) the extract is evaporated to a preselected concentration and
c) the precipitate deposited in step b is separated from the supernatant material,
wherein steps b and c are repeated at least once with the respective supernatant material from step c.

2. Process according to claim 1, characterised in that the ethanolic extract has an ethanol content of at least 40% by volume.

3. Process according to claim 2, characcerised in that the ethanol content is 60 to 90% by volume.

4. Process according to one of claims 1 to 3, in which steps b and c are repeated two to four times.

5. Process according to one of the preceding claims, additionally comprising a step d in which the precipitate obtained in step c is redissolved.

6. Process according to claim 5, in which the redissolution takes place in an ethanol and water mixture with a higher ethanol content than that of the original extraction solvent.

7. Process according to claim 5 or 6, in which the redissolved precipitates are again subjected to one or more sequences of steps b, c and optionally d.

8. Process according to one of the preceding claims, characterised in that the concentration or fractionation in steps b and c is carried out using an Inox-Glatt® apparatus.

9. Process according to claim 8, characterised in that drying is carried out at a forward run or return run temperature between 150 and 100°C or 20 and 1°C, an interior temperature between 10°C and 80°C, a vapour temperature between 15 and 65°C, a pressure between 0.5 and 1000 mbar, a stirrer speed between 0 and 10 rpm and a chopper speed between 200 and 800 rpm.

10. Extract fraction of *Hypericum perforatum L.*, obtainable by one of the processes according to claims 1 to 9.

11. Extract fraction according to claim 10, characterised in that it is tree precipitate or the redissolved precipitate from the first sequence of steps b to c or d, evaporation being carried out to a concentration of 40 to 50% in step b.

12. Extract fraction according to claim 10, characterised in that it is the precipitate from the third step c or the redissolved precipitate from the third step d, the third step taking place from a concentration of 60 to 75% to a concentration of 100%.

13. Extract fraction according to claim 10, characterised in that it is a fraction in which the hypericine content is enriched in relation to the total extract.

14. Extract fraction according to claim 10, characterised in that it is a fraction in which the hyperforine content is enriched in relation to the total extract.

15. Pharmaceutical preparation containing an extract fraction according to one of claims 10 to 14.

16. Pharmaceutical preparation containing an extract fraction according to claim 13, in which the hypericine content is enriched.

17. Pharmaceutical preparation containing an extract fraction according to claim 14, in which the hyperforine content is enriched.

## Revendications

1. Procédé pour l'obtention de fractions d'extraits de Hypericum perforatum L., dans lequel
a) on obtient un extrait éthanolique aqueux de Hypericum perforatum L. selon des procédés connus,
b) on concentre l'extrait à un degré de concentration prédéterminé et
c) on sépare du surnageant le précipité qui s'est déposé à l'étape b.,
dans lequel on répète au moins une fois les étapes b. et c. avec le surnageant respectif de l'étape c.

2. Procédé selon la revendication 1, caractérisé en ce que l'extrait éthanolique présente une concentration en éthanol d'au moins 40% en volume.

3. Procédé selon la revendication 2, caractérisé en ce que la concentration en éthanol va de 60 à 90% en volume.

4. Procédé selon l'une des revendications 1 à 3, dans lequel on répète de deux à quatre fois les étapes b. et c.

5. Procédé selon l'une des revendications précédentes, comprenant en outre une étape d., dans laquelle on redissout le précipité obtenu à l'étape c.

6. Procédé selon la revendication 5, dans lequel la redissolution est effectuée dans un mélange éthanol/eau à teneur en éthanol plus élevée que celle du solvant d'extraction d'origine.

7. Procédé selon la revendication 5 ou 6, dans lequel on réitère une ou plusieurs séries d'étapes b., c. et éventuellement d. sur les précipités redissous.

8. Procédé selon la revendications précédentes, caractérisé en ce que la contraction respectivement la fractionnement des étapes b. et c. est effectué à l'aide d'une installation Inox-Glatt®.

9. Procédé selon la revendication 8, caractérisé en ce que l'on effectue le séchage à une température de tête de distillation, respectivement de reflux, entre 150 et 100°C, respectivement entre 20 et 100°C, une température intérieure entre 10°C et 80°C, une température de vapeur entre 15 et 65°C, sous une pression entre 0,5 et 1000 mbar, une vitesse d'agitation entre 0 et 10 tr/min. et une vitesse de hachage entre 300 et 800 tr/min.

10. Fraction d'extrait de Hypericum perforatum L., que l'on peut obtenir conformément à l'un des procédés selon les revendications 1 à 9.

11. Fraction d'extrait selon la revendication 10, caractérisée en ce qu'il s'agit du précipité ou du précipité redissous de la première série d'étapes b. à c. ou d., dans laquelle on concentre à l'étape b. jusqu'à un degré de concentration de 40 à 50%.

12. Fraction d'extrait selon la revendication 10, caractérisée en ce qu'il s'agit du précipité de la troisième étape c. ou du précipité redissous de la troisième étape d., dans laquelle la troisième étape est effectuée d'un taux de concentration de 60 à 75% à un taux de concentration de 100%.

13. Fraction d'extrait selon la revendication 10, caractérisée en ce qu'il s'agit d'une fraction enrichie en ce qui concerne la teneur en hypéricine par rapport à l'extrait global.

14. Fraction d'extrait selon la revendication 10, caractérisée en ce qu'il s'agit d'une fraction enrichie en ce qui concerne la teneur en hyperforine par rapport à l'extrait global.

15. Préparation pharmaceutique contenant une fraction d'extrait selon l'une des revendications 10 à 14.

16. Préparation pharmaceutique, contenant une fraction d'extrait enrichie en teneur en hypéricine selon la revendication 13.

17. Préparation pharmaceutique , contenant une fraction d'extrait enrichie en teneur en hyperforine selon la revendication 14.
